# EUROPEAN PATENT SPECIFICATION

(11) **EP 1 778 193 B1**
(45) Date of publication and mention of the grant of the patent: **26.12.2007**
(21) Application number: 05768606.5
(22) Date of filing: 27.07.2005
(51) Int. Cl.: A61K 9/16, A61K 31/137

(54) **SUSTAINED RELEASE PHARMACEUTICAL PARTICULATE COMPOSITION COMPRISING VENLAFAXINE**
VENLAFAXIN ENTHALTENDE PHARMAZEUTISCHE TEILCHENFÖRMIGE ZUSAMMENSETZUNG MIT VERZÖGERTER FREISETZUNG
COMPOSITION PHARMACEUTIQUE A LIBERATION PROLONGEE COMPRENANT DE LA VENLAFAXINE

(30) Priority: 28.07.2004 DE 102004036641
(43) Date of publication of application: 02.05.2007
(73) Proprietor: KRKA, tovarna zdravil, d.d., Novo mesto, 8501 Novo mesto (SI)
(72) Inventor: PISEK, Robert, 1000 Ljubljana (SI); ZUPANCIC, Silvo, 8000 Novo mesto (SI); SEGULA, Zakelj, Mojca, 2000 Maribor (SI)
(74) Representative: UEXKÜLL & STOLBERG
(86) International application number: PCT/EP2005/008149
(87) International publication number: WO 2006/010605

(56) References cited:
- WO-A-03/041692
- WO-A-03/103637
- WO-A-20/04047718

## Description

### FIELD OF THE INVENTION

The present invention is directed to a method for the preparation of sustained release pharmaceutical compositions comprising venlafaxine or a salt thereof.

The invention is also directed to a sustained release pharmaceutical composition comprising venlafaxine or a salt thereof, which composition can be in the form of coated pellets or capsules for once-a-day oral administration.

### BACKGROUND OF THE INVENTION

1-[2-Dimethylamino-1-(4-methoxyphenyl)-ethyl]-cyclohexanol, in the following referred to as venlafaxine, has been disclosed in EP 0 112 669. Its hydrochloride salt formulation is currently available under the trade name Effexor®, which is marketed as a racemic mixture, i.e. as a 1:1-mixture of the (+) and (-) enantiomeres of venlafaxine. It is used for the treatment of depression. Presently venlafaxine hydrochloride is admistered to adults as conventional immediate release tablets or as 24 hour extended release multiparticulate capsules.

Venlafaxine hydrochloride has a high solubility in water. It is known in the art that it is very difficult to develop a pharmaceutical composition which exhibits a very slow dissolution rate of a drug having a high solubility. In addition to that venlafaxine hydrochloride is polymorphic. Important properties of polymorphic forms of a drug, i.e. dissolution rate, bioavailability and chemical stability, can differ substantially. Therefore polymorphs of a compound usually differ with regard to their dissolution characteristics and their bulk properties. This has a significant effect on the method of formulating the solid drug and can also affect the bioavailability of the drug. Exactly this is the case with venlafaxine hydrochloride, and therefore the polymorphic form of venlafaxine hydrochloride which is isolated in its synthesis and the one which is used in a formulation must have a defined and preferably the desired crystalline modification.

Sustained release dosage forms are often attractive for the optimisation of therapy not only because the frequency of administration can be reduced, but also because of the reduction of fluctuations in the blood level. A lower maximum level of the drug in the blood can reduce the severity of dose-dependent side effects and thus may improve drug tolerance. Other advantages of reduced dosage regimens for the patients are improved convenience and better assurance of compliance, since sustained release compositions maintain substantially constant blood levels and avoid the fluctuations associated with the conventional immediate release compositions administered more than once a day.

Sustained release formulations of venlafaxine hydrochloride are known in the prior art. EP 0 797 991 and EP 1 028 718 describe coated spheroids of venlafaxine hydrochloride, wherein a spheroid core, prepared by extruding and spheronizing a mixture of the drug with microcrystalline cellulose and hydroxypropylmethyl cellulose (HPMC), is coated with a mixture of ethyl cellulose and hydroxypropylmethyl cellulose.

WO 03/041692 discloses a sustained release composition in which venlafaxine hydrochloride is combined with a binder and sprayed on non pareil inert cores. Such coated cores are then additionally coated with a polymeric layer which enables the controlled release of venlafaxine hydrochloride. Appropriate coating polymers are Eudragit® polymers or cellulose derivatives.

WO 02/102129 describes a method for the preparation of prolonged liberation capsules filled with tri-coated micro granules containing venlafaxine. Spherical micro granules made from sugar and starch are first wetted with povidone solution, powdered with venlafaxine hydrochloride and then coated with ethyl cellulose solution. Ethyl cellulose can at least partially be replaced by Eudragit®, ethers or cellulose esters. This method results in the formation of an inhomogeneous layer of the active ingredient which negatively effects the release of the drug.

US 2003/0190354 describes an extended release composition comprising as active compound venlafaxine hydrochloride in a matrix tablet dosage form, in which venlafaxine is mixed with a combination of hydrophilic, e.g. HPMC, and hydrophobic, e.g. ethyl cellulose, matrix forming components. Kollidon® SR, a polyvinyl acetate based excipient, either in its dry form or as suspension may be used as a binder.

A common problem of the prior art methods for preparing venlafaxine hydrochloride is the change of the polymorphic form of the active ingredient. None of the prior art documents addresses the problem of maintaining the desired polymorphic form of venlafaxine hydrochloride during the preparation of pharmaceutical dosage forms. Maintaining the polymorphic form of the drug is extremely important inter alia in respect of the bioavailability of the drug as has been outlined above.

Therefore, it is an object of the present invention to provide a method for preparing sustained release pharmaceutical composition comprising venlafaxine hydrochloride, wherein the form of venlafaxine is not changed in the course of the process even if the drug comes into contact with solvent, e.g. water, which causes the polymorphic change of the venlafaxine hydrochloride. It is a further object to reduce the number of method steps so that the costs of production are low.

In addition to that it would be highly advantageous, if a composition could be obtained which has such a flexibility that minor impacts do not lead to a damaged composition which consequently could lead to an undesired immediate release of the highly soluble venlafaxine and which compositions also show a self-repair mechanism if damaged. It is also desirable to have compositions which provide a homogeneous release of the drug.

These objects are surprisingly solved by a method for the preparation of a pharmaceutical composition containing venlafaxine or a pharmaceutically acceptable salt thereof comprising the steps of
(1) preparing a suspension of venlafaxine or a venlafaxine salt in a solvent wherein venlafaxine or the venlafaxine salts has a solubility of less than 25 g/l at room temperature;
(2) converting the suspension of step (1) into particulate form;
(3) coating the particles obtained in step (2) with a layer of a sustained release polymer.

The solvent used in step (1) is preferably at least one selected from the group consisting of 1-propanol, 2-propanol, butanol, cyclohexane, diethyl ether, heptane, isopropyl acetate, propyl acetate and toluene. The solvent does preferably not contain water or ethanol since it was found that these solvents favour polymorphic conversions.

It is preferred that the composition comprises venlafaxine in the form of a salt thereof, preferably the hydrochloride, and more preferably as substantially pure or pure polymorphic form. Form I of venlafaxine hydrochloride is defined as in article in Acta Crystallographica (2000) C56, 1009-101*,* various other polymorphs are disclosed in WO 02/45658, WO 02/36542, WO 02/46140, WO 03/042161, WO 03/048082, WO 03/050075 and WO 03/050074. A polymorphic form is considered to be substantially pure if it contains less than 5 % by weight, preferably less than 2 % by weight and most preferably less than 1 % by weight of other polymorphic forms. The most preferred polymorphic form is polymorphic form I.

It is furthermore preferred that the suspension of venlafaxine or the salt thereof in the solvent also comprises a binder. The binder should preferably be soluble in the solvent.

The binder used in step (1) is preferably selected from the group consisting of cellulose, cellulose derivatives, polyvinyl pyrrolidone (PVP) and mixtures thereof. Preferred cellulose derivatives are hydroxypropylmethyl cellulose and/or hydroxypropyl cellulose. In particular, the binder used in step (1) is hydroxypropyl cellulose.

It is preferred that the weight ratio of venlafaxine for venlafaxine salt to the binder in the suspension of step (1) is at least 2 : 1 and up to 20 : 1 and it is even more preferred that the weight ratio ranges from 8 : 1 to 14 : 1, calculated on the basis of venlafaxine base.

The suspension of step (1) can also comprise an antisticking agent. Such an antisticking agent is recommendable if higher concentrations of binder are used. An antisticking agent, such a talc, reduces the sticking tendency and thereby prevents the agglomeration of pellets as well as adhesion effects to the wall during the further processing.

In step (2) the suspension is preferably converted into particulate form by coating the suspension of step (1) onto inert core particles. Thereby sustained release pharmaceutical composition are obtained comprising inert cores, venlafaxine or a salt thereof as an active ingredient in the first layer, and a sustained release polymer in the second layer. The compositions may additionally comprise a separating layer as will be explained in the following.

The inert cores preferably consist substantially of saccharose, starch, microcrystalline cellulose or mixtures thereof, preferably a mixture of saccharose and starch. Non pareil particles are particularly preferred.

It is surprising that the dissolution rate of the pharmaceutical composition according to invention is not affected by the nature nor the amount of the inert core particle material.

The sustained release polymer used in step (3) is preferably a hydrophobic polymer, more preferably a polymer selected from polyvinyl acetate (PVAc) and polyvinyl pyrrolidone (PVP), poly(meth)acrylates such as copolymers of methacrylic acid and/or acrylic acid with (meth)acrylates comprising functional groups such as trimethyl-ammoniumethylmethacrylate, neutral polymers and copolymers of methacrylic acid and acrylic acid, ethylcellulose, methylcellulose and mixtures thereof. Any of these polymers can be used alone, two or more of these polymers can be applied in the form of separate polymer layers or may be applied in the form of a mixture. The sustained release polymers are employed in the form of an aqueous suspension or in the form of a solution in organic solvent.

It is particularly preferred that the sustained release polymer used in step (3) is a combination of PVAc and PVP, either in form of separate polymer layers or in the form of a mixture of these polymers. In was found that combinations of PVAc and PVP provide the compositions with an enormous plasticity. As a result, the compositions and in particular the second layer are very resistant to mechanical stress and show a self-repair mechanism, particularly when a particle coated with this second layer is introduced into an aqueous medium. Because of this self-repair mechanism and the overall stability, the probability of an instantaneous release of the venlafaxine is reduced and therefore the safety of the pharmaceutical composition according to the invention is further enhanced. It is a further benefit that no curing step is necessary to harden these polymers.

Moreover, in step (3) a mixture comprising a sustained release polymer and a plasticizer for further enhancing film formation and its flexibility can be used. Suitable plasticizers are 1,2-propylene glycol, triethyl citrate, polyethylene glycol, dibutyl sebacate and triacetin, with triethyl citrate being the preferred plasticizer.

The sustained release polymer and the optional plasticizer used in step (3) can be further combined with an antisticking agent, such as talc. It is preferred that the weight ratio of antisticking agent to the sustained release polymer, is at least 1:1 and up to 1:10, preferably from 1:2 to 1:5.

The method of the present invention can further advantageously comprise the additional step of applying a separating layer on the compositions formed in step (2). The separating layer is preferably formed by applying an aqueous suspension or an organic solution of a suitable polymer which is preferably selected from the group consisting of cellulose and cellulose derivatives, polyvinylpyrrolidone, polyvinyl alcohol (PVA) and mixtures thereof. Preferred cellulose derivatives are hydroxypropylcellulose and hydroxypropylmethylcellulose. Besides these polymers the suspension or solution for applying the separating layer can advantageously comprise an antisticking agent, such as talc.

In a preferred embodiment, the process of the invention thus results in the formation of a pharmaceutical composition comprising:
(a) inert cores,
(b) a first layer, which comprises venlafaxine and optionally also a binder and which is layered on inert cores (a), and
(c) a second layer, which comprises the sustained release polymer and optionally binder and plasticizer, which layer is coated on layer (b) or optional layer (d) and
(d) optionally a separating layer, which is coated on layer (b), i.e. is arranged between layer (b) and layer (c).

Accordingly, the composition is preferably in the form of particles, such as pellets, spheroids or granules. The discrete coated particles can be filled into larger units, such as capsules or can be formed into tablets.

Each of the layers is preferably dried before applying the next layer.

The inert cores can be coated with the first and the second layer and optionally the separating layer using a fluidised bed or coating pan. Preferably a fluidised bed coater is used, e.g. a bottom, a tangential or a top-spray coater. A fluidised bed coater, e.g. from Wurster or Huettlin, is a system in which an air jet which is injected from underneath the inert cores fluidises the same and effects the drying of the first and second layer after being sprayed on the cores. The preferred method for the manufacture of this composition is advantageous, because both phases of the method, i.e. coating and drying and the forming of the first and the second layer, can take place in the same container. By this simple arrangement, the reproducibility of the method and thereby the quality of the resulting pharmaceutical composition is excellent.

Optionally, particles differing in the thickness of the first and/or the second layer are combined before being filled into larger units or formed into tablets. By combining particles, such as pellets, which differ in the thickness of their second layer the release profile can be tailored as desired.

It has surprisingly been found that the polymorphic form of venlafaxine hydrochloride is not changed in the process of the present invention, even if water containing suspensions are used for applying the sustained release polymer or for the application of the separating layer. Moreover, the process of the invention results in the formation of a very uniform layer comprising the active ingredient.

Pharmaceutical compositions obtainable by the process of the present invention are also subject of the present invention. These compositions preferably have the form of particles, pellets, spheroids, granules, tablets or capsules.

The composition preferably comprises venlafaxine or a venlafaxine salt in an amount of 15 to 50 wt %, preferably 20 to 45 wt % and most preferred of 22 to 37 wt %, calculated as the base form. By the term "calculated as the base form" is meant that the amount of venlafaxine which is not present in the free base form, e.g. in form of the preferred hydrochloride form, has to be converted into the corresponding amount of the free base form. For example a pharmaceutical composition which comprises 50 mg of the preferred venlafaxine hydrochloride, which has a molar mass of 330.87 g/mol, would be regarded as 41.92 mg of venlafaxine, which has a molar mass of 277.40 g/mol.

In addition to the active ingredient the first layer preferably also comprises a binder. The binder is preferably added in such an amount that the final preparation contains 2 to 5 wt-% such as 2 to 4.5 wt-%, more preferably 3 to 5 wt-% such as 3 to 4.5 wt-%, and most preferred 3 to 4 wt-% of the binder, based on the total weight of the pharmaceutical composition.

The composition of the invention preferably comprise the sustained release polymer in an amount of 2 to 35 wt %, more preferably 3 to 30 wt %, and most preferably 4 to 25 wt %, based on the total weight of the composition.

The release rate of venlafaxine from the composition of the present invention decreases with increasing thickness of the second layer comprising the sustained release polymer. The release rate is the amount of venlafaxine which is liberated from the composition in a given period of time. Initially, water has to penetrate the second layer and enter the particle or tablet in order to at least partially dissolve the venlafaxine before the venlafaxine can diffuse through the second layer.

Besides the release rate, also the lag time is influenced by the thickness of the second layer. The lag time is the time between first contact of the pharmaceutical composition with water and the first measurable release of venlafaxine. With increasing thickness of the second layer the lag time is also increased.

It is preferred that the amount of plasticizer in the second layer ranges from 0 to 20 wt %, based on the dry weight of the sustained release polymer.

The pharmaceutical compositions of the present invention comprise inert cores in an amount of preferably 20 to 55 wt %, more preferably 25 to 50 wt %, and most preferably 30 to 45 wt %, based on the total weight of the pharmaceutical composition.

If present, the polymer(s) of the separating layer preferably comprises 1 to 5 wt-% of the pharmaceutical composition. In addition the separating layer can comprise an antisticking agent, preferably in an amount of 0.1 to 5 wt.-%, based on the total weight of the pharmaceutical composition.

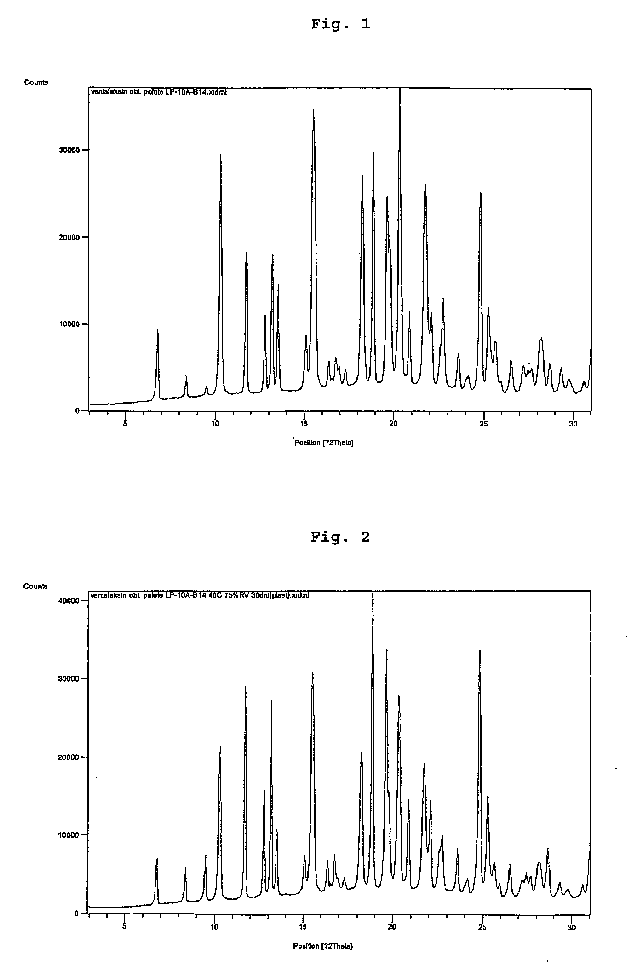

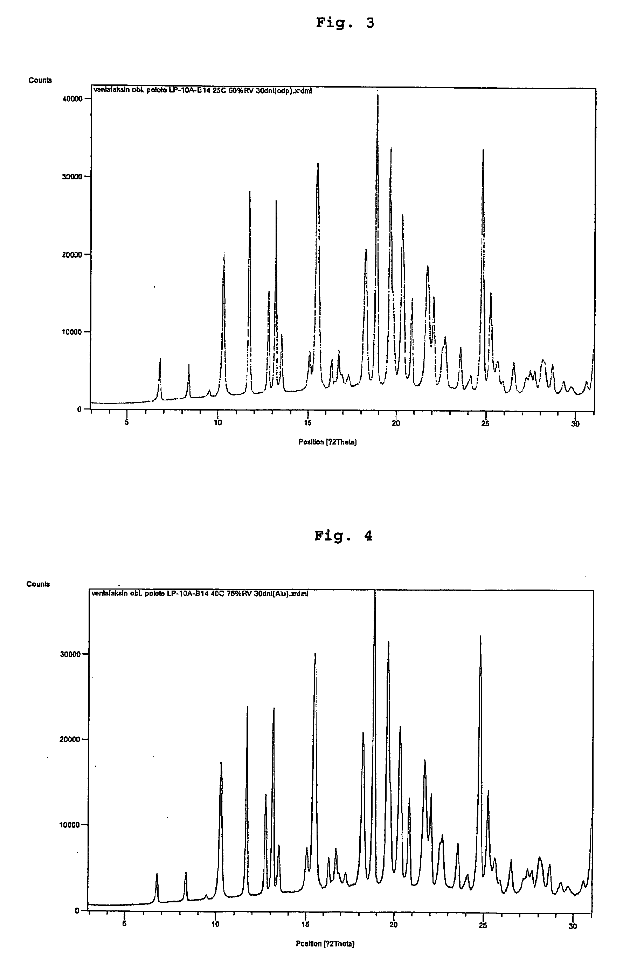

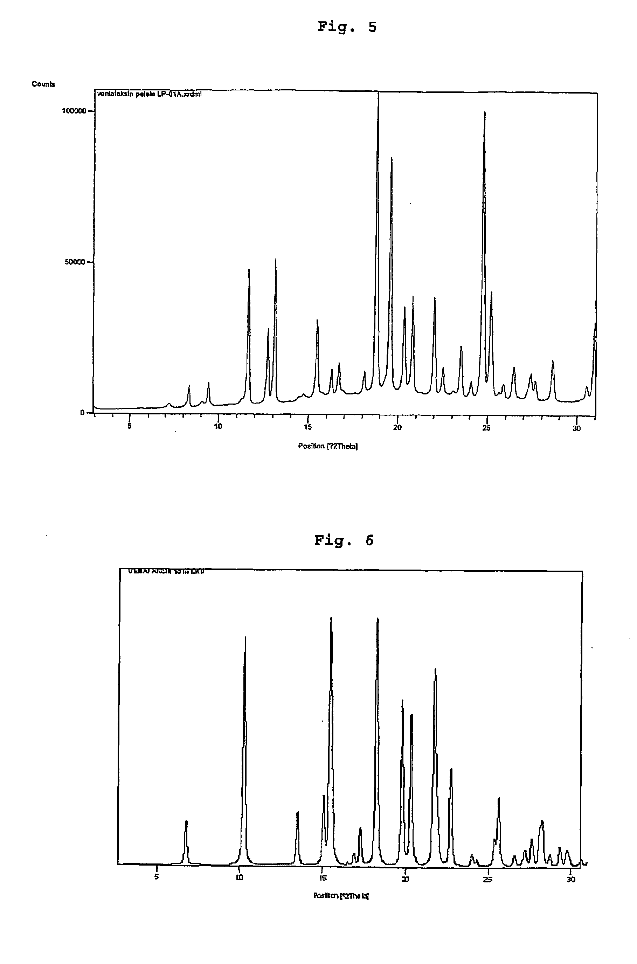

The maintenance of polymorphic form I during the method of the present invention is illustrated by Figures 1 to 6.
Figure 1 is an XRPD spectrum of pellets produced in Example 1 immediately after production;
Figure 2 is an XRPD spectrum of pellets produced in Example 1 measured after 1 month storage in a plastic container with desiccant at 40°C and 75% RH;
Figure 3 is an XRPD spectrum of pellets produced in Example 1 measured after 1 month storage in an open plastic container at 25°C and 60% RH;
Figure 4 is an XRPD spectrum of pellets produced in Example 1 measured after 1 month storage in Alu/Alu packaging at 40°C and 75% RH;
Figure 5 is an XRPD spectrum of pellets produced in Example 4 immediately after production; and
Figure 6 is an XRPD spectrum of polymorphic form I of venlafaxine

The invention is further illustrated by the following examples:

### Examples:

### Example 1: LP-10A-B14

89.6 g of hydroxypropyl cellulose were dissolved in 1781.85 g of 1-propanol. 1187.90 g of venlafaxine hydrochloride were added to the resulting solution and the dispersion was homogenised in an appropriate mixer unit until a substantially homogeneous dispersion was obtained. This dispersion was then sprayed onto 1260 g of inert cores, i.e. non pareil seeds comprising a mixture of saccharose and starch, in a Wurster fluidised bed equipment to form cores with a first layer.

1214.4 g of the coated cores were additionally coated in the Wurster fluidised bed equipment with 568.4 g of a 30% by weight aqueous dispersion of PVAc and PVP, 17.2 g of triethylcitrate, 60.9 g of talc and 568.4 g of purified water.

The samples were stored 1 month at different conditions in:
- Plastic container with drying unit at 40°C and 75% RH
- Open plastic container at 25°C and 60% RH
- Alu/Alu packaging at 40°C and 75% RH

### Example 2: LP-17A-NE30

124.80 g of hydroxypropylcellulose were dissolved in 2481.86 g of 1-propanol. 1654.58 g of venlafaxine hydrochloride were added to the resulting solution and the suspension was homogenised in an appropriate mixer unit a substantially homogeneous suspension was obtained. This suspension was then sprayed onto 1755.00 g of inert cores, i.e. non pareil seeds comprising a mixture of saccharose and starch, in a Wurster fluidised bed equipment to form cores with a first layer.

1305.00 g of the coated cores were additionally coated in the Wurster fluidised bed equipment with 978.75 g of Eudragit NE 40 D, 391.50 g of talc and 1436.81 g of purified water.

### Example 3: LP-15A-V-E30

115.20 g of hydroxypropylcellulose were dissolved in 2290.95 g of 1-propanol. 1527.30 g of venlafaxine hydrochloride were added to the resulting solution and the suspension was homogenised in an appropriate mixer unit until a substantially homogeneous suspension was obtained. This suspension was then sprayed onto 1620.00 g of inert cores, i.e. non pareil seeds comprising a mixture of saccharose and starch, in a Wurster fluidised bed equipment to form cores with a first layer.

1540.63 g of the coated cores were additionally coated in the Wurster fluidised bed equipment with 46.22 g of hydroxypropylcellulose, 52.30 g of talc and 1000.00 g of 1-propanol.

1542.73 g of the coated cores were additionally coated in the Wurster fluidised bed equipment with 1542.73 g of Eudragit RS 30 D, 92.56 g of triethylcitrate, 231.41 g of talc and 2005.55 g of purified water

### Example 4: LP-01A:

525 g of venlafaxine hydrochloride were dissolved in 918 g of purified water. 72 g of talk were added to the resulting solution and the suspension was homogenised in an appropriate mixer unit until a substantially homogeneous suspension was obtained. This suspension was then sprayed onto 1225 g of inert cores, i.e. non pareil seeds comprising a mixture of saccharose and starch, in a Wurster fluidised bed equipment to form cores with a first layer.

### Example 5: LP-21 A-V-B7

128.00 g of hydroxypropylcellulose were dissolved in 2545.50 g of 1-propanol. 1697.00 g of venlafaxine hydrochloride were added to the resulting solution and the suspension was homogenised in an appropriate mixer unit until a substantially homogeneous suspension was obtained. This suspension was then sprayed onto 1500.00 g of inert cores, i.e. non pareil seeds comprising a mixture of saccharose and starch, in a Wurster fluidised bed equipment to form cores with a first layer.

2992.50 g of the coated cores were additionally coated in the Wurster fluidised bed equipment with a solution of 74.81 g of polyvinylpyrrolidone in 1197.00 g of ethanol.

2931.00 g of the coated cores were additionally coated in the Wurster fluidised bed equipment with a mixture of 205.17 g of ethylcellulose, 20.52 g of dibutyl sebacate and 3385.00 g of ethanol.

## Claims

1. A method for the preparation of a pharmaceutical composition containing venlafaxine or a pharmaceutically acceptable salt thereof comprising the steps of
(1) preparing a suspension of venlafaxine or a venlafaxine salt in a solvent wherein venlafaxine or the venlafaxine salt has a solubility of less than 25 g/l at room temperature;
(2) converting the suspension of step (1) into particulate form;
(3) coating the particles obtained in step (2) with a layer of a sustained release polymer.

2. The method of claim 1, wherein venlafaxine hydrochloride is used as the active ingredient.

3. The method of claim 2, wherein venlafaxine hydrochloride is used in the form of a substantially pure polymorph.

4. The method of any one of the preceding claims, wherein the suspension of step (1) additionally comprises a binder.

5. The method of claim 4, wherein a binder is used which is soluble in the solvent.

6. The method of claim 5, wherein the binder is selected from the group consisting of cellulose derivatives, polyvinyl pyrrolidone (PVP), polyvinyl alcohol (PVA) and mixtures thereof.

7. The method of any one of claims 4 to 6, wherein venlafaxine or the salt thereof and the binder are used in a weight ratio of 2:1 to 20:1, calculated on the basis of venlafaxine base.

8. The method of any one of the preceding claims, wherein the suspension of step (1) additionally comprises an anti-sticking agent.

9. The method of claim 8, wherein the anti-sticking agent is talc.

10. The method of any one of the preceding claims, wherein in step (2) the suspension is converted into particulate form by coating the suspension of step (1) onto inert core particles.

11. The method of claim 10, wherein the inert cores essentially consist of saccharose, starch, microcrystalline cellulose or a mixture thereof, in particular a mixture of saccharose and starch.

12. The method of claim 10 or 11, wherein the inert core particles are non pareil particles.

13. The method of any one of claims 1 to 12 wherein the sustained release polymer is a hydrophobic sustained release polymer.

14. The method of claim 13 wherein the sustained release polymer is selected from the group consisting of polyvinyl acetate (PVAc), polyvinyl pyrrolidone (PVP), poly(meth)arylates such as copolymers of acrylic acid and/or methacrylic acid with trimethylammoniumethylmetacrylate, polymers and copolymers of methacrylic acid and acrylic acid, ethylcellulose, methylcellulose and mixtures thereof.

15. The method of claim 14, wherein a polymer mixture is used in step (3) which comprises a mixture of polyvinyl acetate (PVAc) and polyvinyl pyrrolidone (PVP).

16. The method of any one of claims 1 to 15, wherein in step (3) a mixture of a sustained release polymer and a plasticizer is used.

17. The method of claim 16, wherein the plasticizer is selected from the group consisting of 1,-2-propylene glycol, triethyl citrate, polyethylene glycol, triacetin, dibutyl sebacate and mixtures thereof.

18. The method of any one of claims 1 to 17 comprising the additional step of applying a separating layer on the particles formed in step (2).

19. The method of claim 18, wherein the separating layer is formed by applying an aqueous suspension or an organic solution of a polymer which is selected from the group consisting of cellulose and cellulose derivatives, polyvinylpyrrolidone, polyvinyl alcohol (PVA) and mixtures thereof.

20. The method of any one of claims 1 to 19 additionally comprising the drying of the particles obtained in step (2), the coated particles obtained in step (3) and/or the particles obtained in the method of claim 18 and 19.

21. The method of any one of claims 1 to 20 wherein the method is performed in a fluid bed coater.

22. The method of any one of claims 10 to 21, comprising coating the suspension of step (1) onto inert core particles in order to obtain a lot of particles comprising a layer formed by depositing the suspension of step (1) having a first thickness, coating the suspension of step (1) onto inert core particles in order to obtain at least one lot of particles comprising a layer formed by depositing the suspension of step (1) having a different thickness, and mixing the lots of particles in order to obtain a lot of particles differing in the thickness of the layer formed by depositing the suspension of step (1).

23. The method of any one of claims 1 to 22, wherein the particles obtained in step (3) are filled into capsules or formed into tablets.

24. A pharmaceutical composition obtainable by the process of any one of claims 1 to 23.

25. The composition of claim 24, comprising 15 to 50 wt.-% venlafaxine or venlafaxine salt, calculated on the basis of venlafaxine base and based on the total weight of the composition.

26. The composition of claim 24 or 25, comprising 2 to 35 wt.-% sustained release polymer, based on the total weight of the composition.

27. The composition of any one of claims 24 to 26 comprising 20 to 55 wt-% inert cores, based of the total weight of the composition.

28. The composition of any one of claims 24 to 27, wherein the venlafaxine or venlafaxine salt containing layer comprises 2 to 5 wt.-% of a binder, based of the total weight of the composition.

29. The composition of any one of claims 24 to 28, wherein the polymer of the separating layer comprises 1 to 5 wt.-% of the total weight of the composition.

## Patentansprüche

1. Verfahren zur Herstellung einer pharmazeutischen Zusammensetzung, die Venlafaxin oder ein pharmazeutisch annehmbares Salz davon enthält, das die folgenden Stufen aufweist:
(1) Herstellen einer Suspension von Venlafaxin oder einem Venlafaxinsalz in einem Lösungsmittel, wobei Venlafaxin oder das Venlafaxinsalz eine Löslichkeit von weniger als 25 g/l bei Raumtemperatur hat;
(2) Umwandeln der Suspension von Stufe (1) in Teilchenform;
(3) Beschichten der in Stufe (2) erhaltenen Teilchen mit einer Schicht eines Polymers für verzögerte Freisetzung.

2. Verfahren nach Anspruch 1, bei dem als aktiver Bestandteil Venlafaxinhydrochlorid verwendet wird.

3. Verfahren nach Anspruch 2, bei dem Venlafaxinhydrochlorid in Form eines im Wesentlichen reinen Polymorphs verwendet wird.

4. Verfahren nach einem der vorhergehenden Ansprüche, bei dem die Suspension von Stufe (1) zusätzlich ein Bindemittel enthält.

5. Verfahren nach Anspruch 4, bei dem ein Bindemittel verwendet wird, das in dem Lösungsmittel löslich ist.

6. Verfahren nach Anspruch 5, bei dem das Bindemittel aus der Gruppe bestehend aus Cellulosederivaten, Polyvinylpyrrolidon (PVP), Polyvinylalkohol (PVA) und Mischungen davon ausgewählt ist.

7. Verfahren nach einem der Ansprüche 4 bis 6, bei dem Venlafaxin oder das Salz davon und das Bindemittel in einem Gewichtsverhältnis von 2:1 bis 20:1 verwendet werden, berechnet auf Basis von Venlafaxin-Base.

8. Verfahren nach einem der vorhergehenden Ansprüche, bei dem die Suspension von Stufe (1) zusätzlich ein Antiverklebungsmittel enthält.

9. Verfahren nach Anspruch 8, bei dem das Antiverklebungsmittel Talkum ist.

10. Verfahren nach einem der vorhergehenden Ansprüche, bei dem die Suspension in Stufe (2) in Teilchenform umgewandelt wird, indem die Suspension von Stufe (1) als Beschichtung auf inerte Kernteilchen aufgebracht wird.

11. Verfahren nach Anspruch 10, bei dem die inerten Kerne im Wesentlichen aus Saccharose, Stärke, mikrokristalliner Cellulose oder einer Mischung davon bestehen, insbesondere aus einer Mischung aus Saccharose und Stärke.

12. Verfahren nach Anspruch 10 oder 11, bei dem die inerten Kernteilchen Nonpareil-Teilchen sind.

13. Verfahren nach einem der Ansprüche 1 bis 12, bei dem das Polymer für verzögerte Freisetzung ein hydrophobes Polymer für verzögerte Freisetzung ist.

14. Verfahren nach Anspruch 13, bei dem das Polymer für verzögerte Freisetzung aus der Gruppe bestehend aus Polyvinylacetat (PVAc), Polyvinylpyrrolidon (PVP), Poly(meth)acrylaten, wie Copolymeren von Acrylsäure und/oder Methacrylsäure mit Trimethylammoniumethylmethacrylat, Polymeren und Copolymeren von Methacrylsäure und Acrylsäure, Ethylcellulose, Methylcellulose und Mischungen davon ausgewählt ist.

15. Verfahren nach Anspruch 14, bei dem in Stufe (3) eine Polymermischung verwendet wird, die eine Mischung aus Polyvinylacetat (PVAc) und Polyvinylpyrrolidon (PVP) enthält.

16. Verfahren nach einem der Ansprüche 1 bis 15, bei dem in Stufe (3) eine Mischung aus einem Polymer für verzögerte Freisetzung und einem Weichmacher verwendet wird.

17. Verfahren nach Anspruch 16, bei dem der Weichmacher aus der Gruppe bestehend aus 1,2-Propylenglykol, Triethylcitrat, Polyethylenglykol, Triacetin, Dibutylsebacat und Mischungen davon ausgewählt ist.

18. Verfahren nach einem der Ansprüche 1 bis 17, bei dem in einer weiteren Stufe eine Trennschicht auf die in Stufe (2) gebildeten Teilchen aufgebracht wird.

19. Verfahren nach Anspruch 18, bei dem die Trennschicht gebildet wird, indem eine wässrige Suspension oder eine organische Lösung eines Polymers ausgewählt aus der Gruppe bestehend aus Cellulose und Cellulosederivaten, Polyvinylpyrrolidon, Polyvinylalkohol (PVA) und Mischungen davon aufgebracht wird.

20. Verfahren nach einem der Ansprüche 1 bis 19, bei dem zusätzlich die in Stufe (2) erhaltenen Teilchen, die in Stufe (3) erhaltenen beschichteten Teilchen und/oder die in dem Verfahren von Anspruch 18 oder 19 erhaltenen Teilchen getrocknet werden.

21. Verfahren nach einem der Ansprüche 1 bis 20, bei dem das Verfahren in einem Wirbelbettbeschichter durchgeführt wird.

22. Verfahren nach einem der Ansprüche 10 bis 21, bei dem die Suspension von Stufe (1) als Beschichtung auf inerte Kernteilchen aufgebracht wird, um eine Charge Teilchen zu erhalten, die eine durch Absetzen der Suspension von Stufe (1) gebildete Schicht mit einer ersten Dicke enthalten, die Suspension von Stufe (1) als Beschichtung auf inerte Kernteilchen aufgebracht wird, um mindestens eine Charge Teilchen zu erhalten, die eine durch Absetzen der Suspension von Stufe (1) gebildete Schicht mit anderer Dicke enthalten, und die Chargen der Teilchen gemischt werden, um eine Charge Teilchen zu erhalten, die sich in der Dicke der durch Absetzen der Suspension von Stufe (1) gebildeten Schicht unterscheiden.

23. Verfahren nach einem der Ansprüche 1 bis 22, bei dem die in Stufe (3) erhaltenen Teilchen in Kapseln gefüllt oder zu Tabletten geformt werden.

24. Pharmazeutische Zusammensetzung, die nach dem Verfahren gemäß einem der Ansprüche 1 bis 23 erhältlich ist.

25. Zusammensetzung nach Anspruch 24, die 15 bis 50 Gew.-% Venlafaxin oder Venlafaxinsalz enthält, berechnet auf der Basis von Venlafaxin-Base und bezogen auf das Gesamtgewicht der Zusammensetzung.

26. Zusammensetzung nach Anspruch 24 oder 25, die 2 bis 35 Gew.-% Polymer für verzögerte Freisetzung enthält, bezogen auf das Gesamtgewicht der Zusammensetzung.

27. Zusammensetzung nach einem der Ansprüche 24 bis 26, die 20 bis 55 Gew.-% inerte Kerne enthält, bezogen auf das Gesamtgewicht der Zusammensetzung.

28. Zusammensetzung nach einem der Ansprüche 24 bis 27, bei der die Venlafaxin oder Venlafaxinsalz enthaltende Schicht 2 bis 5 Gew.-% eines Bindemittels enthält, bezogen auf das Gewicht der Zusammensetzung.

29. Zusammensetzung nach einem der Ansprüche 24 bis 28, bei der das Polymer der Trennschicht 1 bis 5 Gew.-% des Gesamtgewichts der Zusammensetzung ausmacht.

## Revendications

1. Procédé de préparation d'une composition pharmaceutique contenant de la venlafaxine ou l'un de ses sels pharmacologiquement admissibles, lequel procédé comporte les étapes suivantes :
1) préparer une suspension de venlafaxine ou d'un sel de venlafaxine, dans un solvant dans lequel la solubilité de la venlafaxine ou de ses sels est inférieure à 25 g/L à température ambiante ;
2) convertir la suspension issue de l'étape (1) en une forme en particules ;
3) et enrober les particules obtenues dans l'étape (2) d'une couche de polymère pour libération retardée.

2. Procédé conforme à la revendication 1, dans lequel on utilise du chlorhydrate de venlafaxine comme ingrédient actif.

3. Procédé conforme à la revendication 2, dans lequel on utilise du chlorhydrate de venlafaxine sous forme polymorphe pratiquement pure.

4. Procédé conforme à l'une des revendications précédentes, dans lequel la suspension de l'étape (1) comprend en outre un liant.

5. Procédé conforme à la revendication 4, dans lequel on utilise un liant qui est soluble dans le solvant.

6. Procédé conforme à la revendication 5, dans lequel le liant est choisi dans l'ensemble constitué par les dérivés de cellulose, la poly(vinylpyrrolidone) (PVP) et le poly(alcool vinylique) (PVA), ainsi que leurs mélanges.

7. Procédé conforme à l'une des revendications 4 à 6, dans lequel la venlafaxine ou son sel et le liant sont employés en un rapport pondéral de 2/1 à 20/1, rapport calculé sur la base de la venlafaxine base.

8. Procédé conforme à l'une des revendications précédentes, dans lequel la suspension de l'étape (1) comprend en outre un agent anti-collant.

9. Procédé conforme à la revendication 8, dans lequel l'agent anti-collant est du talc.

10. Procédé conforme à l'une des revendications précédentes, dans lequel, dans l'étape (2), on convertit la suspension en une forme en particules en enrobant avec la suspension issue de l'étape (1) des particules constituant des noyaux inertes.

11. Procédé conforme à la revendication 10, dans lequel les noyaux inertes sont essentiellement constitués de saccharose, d'amidon ou de cellulose microcristalline, ou d'un mélange de ces corps, en particulier d'un mélange de saccharose et d'amidon.

12. Procédé conforme à la revendication 10 ou 11, dans lequel les particules constituant des noyaux inertes sont des particules de type nonpareille.

13. Procédé conforme à l'une des revendications 1 à 12, dans lequel le polymère pour libération retardée est un polymère hydrophobe pour libération retardée.

14. Procédé conforme à la revendication 13, dans lequel le polymère pour libération retardée est choisi dans l'ensemble formé par le poly(acétate de vinyle) (PVAc), la poly(vinyl-pyrrolidone) (PVP), les polyacrylates et polyméthacrylates comme les copolymères d'acide acrylique et/ou d'acide méthacrylique et de méthacrylate de triméthylammoniométhyle, les polymères et copolymères d'acide méthacrylique et d'acide acrylique, l'éthyl-cellulose et la méthyl-cellulose, ainsi que leurs mélanges.

15. Procédé conforme à la revendication 14, dans lequel on utilise, dans l'étape (3), un mélange de polymères qui comprend un mélange de poly(acétate de vinyle (PVAc) et de poly(vinyl-pyrrolidone) (PVP).

16. Procédé conforme à l'une des revendications 1 à 15, dans lequel on utilise, dans l'étape (3), un mélange d'un polymère pour libération retardée et d'un plastifiant.

17. Procédé conforme à la revendication 16, dans lequel le plastifiant est choisi dans l'ensemble formé par le 1,2-propylèneglycol, le citrate de triéthyle, un polyéthylèneglycol, la triacétine et le sébaçate de dibutyle, ainsi que leurs mélanges.

18. Procédé conforme à l'une des revendications 1 à 17, qui comporte une étape supplémentaire où l'on applique une couche de séparation sur les particules formées lors de l'étape (2).

19. Procédé conforme à la revendication 18, dans lequel on forme la couche de séparation en enrobant les particules d'une suspension aqueuse ou d'une solution, dans un solvant organique, d'un polymère choisi dans l'ensemble formé par la cellulose et les dérivés de cellulose, la poly(vinylpyrrolidone) et le poly(alcool vinylique) (PVA), ainsi que leurs mélanges.

20. Procédé conforme à l'une des revendications 1 à 19, qui comporte en outre le fait de faire sécher les particules obtenues lors de l'étape (2), les particules enrobées obtenues lors de l'étape (3), et/ou les particules obtenues dans une variante de procédé conforme à l'une des revendications 18 et 19.

21. Procédé conforme à l'une des revendications 1 à 20, lequel procédé est mis en oeuvre dans un appareil d'enrobage en lit fluidisé.

22. Procédé conforme à l'une des revendications 10 à 21, qui comporte le fait d'enrober avec la suspension issue de l'étape (1) des particules constituant des noyaux inertes, de manière à obtenir un lot de particules qui comportent une couche formée par dépôt de la suspension issue de l'étape (1) et dotée d'une première épaisseur, le fait d'enrober avec la suspension issue de l'étape (1) des particules constituant des noyaux inertes, de manière à obtenir au moins un lot de particules qui comportent une couche formée par dépôt de la suspension issue de l'étape (1) et dotée d'une épaisseur différente, et le fait de mélanger ces lots de particules pour obtenir un lot de particules qui diffèrent par l'épaisseur de la couche formée par dépôt de la suspension issue de l'étape (1).

23. Procédé conforme à l'une des revendications 1 à 22, dans lequel on met dans des gélules les particules obtenues lors de l'étape (3), ou bien l'on en fait des comprimés.

24. Composition pharmaceutique accessible par un procédé conforme à l'une des revendications 1 à 23.

25. Composition conforme à la revendication 24, qui comprend 15 à 50 % en poids de venlafaxine ou de sel de venlafaxine, pourcentage calculé sur la base de la venlafaxine base et rapporté au poids total de la composition.

26. Composition conforme à la revendication 24 ou 25, qui comprend 2 à 35 % en poids de polymère pour libération retardée, pourcentage rapporté au poids total de la composition.

27. Composition conforme à l'une des revendications 24 à 26, qui comprend 20 à 55 % en poids de noyaux inertes, pourcentage rapporté au poids total de la composition.

28. Composition conforme à l'une des revendications 24 à 27, dans laquelle la couche contenant la venlafaxine ou le sel de venlafaxine comprend 2 à 5 % en poids d'un liant, pourcentage rapporté au poids total de la composition.

29. Composition conforme à l'une des revendications 24 à 28, dans laquelle le polymère de la couche de séparation représente 1 à 5 % du poids total de la composition.
